# EUROPEAN PATENT APPLICATION

(11) **EP 2 407 034 A1**
(43) Date of publication of application: **18.01.2012**
(21) Application number: 10007431.9
(22) Date of filing: 17.07.2010
(51) Int. Cl.: A23L 1/00, A23L 1/29, A23L 1/30, A23P 1/04, A61P 3/04

(54) **Particles**

(71) Applicant: Cognis IP Management GmbH, 40589 Düsseldorf (DE)
(72) Inventor: Müller, Michael, 89165 Dietenheim (DE); Bell, Doris, 40627 Düsseldorf (DE); Horlacher, Peter, 89287 Bellenberg (DE)

(57) **Abstract**

The present invention relates to particles comprising at least one polymer selected from the group consisting of cellulose, derivatives of cellulose and shellac and at least one nutrient selected from the group consisting of fatty acids, monoacylglycerols, diacylglycerols, triacylglycerols, proteins and carbohydrates, wherein the at least one nutrient is coated by the at least one polymer or wherein the at least one nutrient is matrix-encapsulated by the at least one polymer and it relates to a process for making these particles and it relates to the therapeutic use and to the non-therapeutic use of these particles.

## Description

The present invention relates to particles comprising at least one polymer selected from the group consisting of cellulose, derivatives of cellulose and shellac and at least one nutrient selected from the group consisting of fatty acids, monoacylglycerols, diacylglycerols, triacylglycerols, proteins and carbohydrates, wherein the at least one nutrient is coated by the at least one polymer or wherein the at least one nutrient is matrix-encapsulated by the at least one polymer and it relates to a process for making these particles and it relates to the therapeutic use and to the non-therapeutic use of these particles.

Substances or compositions that are used to reduce appetite of humans and/or to increase satiety of humans are known. These substances or compositions can be used to reduce the weight of humans either for therapeutic (including prophylactic) reasons or for non-therapeutic reasons, e. g. for cosmetic reasons or simply because an individual human wants to reduce his or her weight. Increasing satiety leads to reducing appetite.

WO 2006/117069 A1 describes edible emulsions comprising 15 -70 % oil having a saturated fatty acid content of at least 20 %. The emulsions can be added to food matrices whereby a satiation effect is achieved.

WO 2008/066303 discloses food compositions comprising proteins as active ingredient for reducing hunger.

WO 2004/057982 describes the use of special triacylglycerols that retard the onset of hunger if consumed.

Furthermore it is known that if nutrients are directly delivered to the distal small intestine (ileum) to a certain amount they stimulate the secretion of GLP-1 and cause the so called "ileal brake". This is descried in several publications where the nutrient is delivered directly to the ileum by means of an infusion: L. Pirony et al., Gastroenterology 1993; 105: 733-739 **and** J. Keller at al., Am J Physiol Gastrontest Liver Physiol 2006; 290: G704-G709**.**

The impact of carbohydrates on secretion of GLP-1 is described in Shima, K., et al. (1990); Acta Endocrinol (Copenh.); 464-470**.**

The impact of chlorogenic acid on secretion of GLP-1 is described in McCarty MF. , Med Hypotheses. 2005;64(4):848-53. Apart from coffee, chlorogenic acid can be found in the extracts of the following plants: Carrots, bamboo, aster and Disacus.

The impact of Grifolic" an "Grifolic Acid " is described in Hara T et al Naunyn Schmiedebergs Arch Pharmacol. 2009; 380(3):247-55**.** Grifolin can be found in extracts of mushroom Albatrellus.

The ileal brake is a feedback mechanism influencing the digestive process and human appetite/satiation in such a manner that overall food intake is reduced. It is also known that the human peptide GLP-1 is a mediator of the ileal brake. GLP-1 is secreted by L-cells in the ileum, predominately in response to fats and carbohydrates. Nutrients that stimulate the secretion of GLP-1 and therefore activate the ileal brake when reaching the ileum are fatty acids, mono-, di- and triacylglycerols, proteins and carbohydrates, whereby especially fatty acids and their acylglycerols proved to be especially potent.

Mono-, di- and triacylglycerols are mono-, di- and triacylglycerols of fatty acids.

The problem underlying the present invention is to provide substances that can be used to increase satiety of humans.

This problem is solved by the particles according to the claims of the present invention.

Nutrients are disintegrated and absorbed in the stomach and the first section of the small intestine with the help of enzymes and bile. Triacylglycerols are hydrolyzed by lipase enzymes and absorbed before they can reach the ileum. Therefore nutrients must be protected from this digestion process in such a way that they can reach the ileum where they can cause the ileal brake if it is intended to use nutrients in order to cause the ileal brake and if these nutrients shall be administered orally.

Existing weight management formulations mainly describe emulsions or other formulations that provide no or insufficient protection from the digestion process taking place in the stomach and the first section of the small intestine.

The particles according to the present invention endure conditions that exist in the stomach and even retard the release in the intestine. With the help of these particles GLP-1 stimulating actives that are usually digested and/or absorbed can be delivered to the ileum. Thus the portion of nutrients of a weight management formulation that reach the ileum to activate the ileal brake is increased significantly compared to orally administering nutrients that have not been incorporated into particles according to the present invention.

The nutrients according to the present invention are protected by polymers that provide adequate resistance to gastro-intestinal juice. This is achieved by a polymer coating or by a matrix encapsulation in such a way that the release of nutrients in gastro-intestinal juice is retarded in a favored manner compared to the non-protected nutrients.

In one embodiment of the present invention the particles are powder-particles or granules that can be produced by different technologies.

In one embodiment of the present invention the nutrient is oleic acid.

In one embodiment of the present invention the nutrient is oleic acid monoglyceride (monooleyl glycerol).

In one embodiment of the present invention the nutrient is a mixture of different dietary oils comprising oleic acid or oleic acid monoglyceride and other medium or long-chain mono- or polyunsaturated fatty acids or their monoglycerides. In this context medium chain means 10 to 14 C-atoms in a fatty acid, long chain means more than 14 C-atoms in a fatty acid.

In one embodiment of the present invention the nutrient is a carbohydrate selected from the group consisting of a monosaccharide, a disaccharide, an oligosaccharide and a polysaccharide.

In one embodiment of the present invention the particles furthermore comprise at least one plant extract. Preferred plant extracts are selected from the group consisting of an extract from Ipomoea purga, Teucrium scorodonia, Pyrus communis, Ocimum basilicum, Thymus vulgaris, Verbena officinalis, Artemisia dracunculus, Thymus serpyllum, Origanum vulgare, Stachys officinalis, Arctium lappa, Helianthus annuus, Malus domestica, Cichorium intybus, Salvia spec., Solanum tuberosum, Raphanus sativus, Brassica oleracea, Citrus paradise, Citrus sinensis, Citrus limon, Fragaria spec., Capsicum frutescens, Allium sativum, Arachis hypogaea, Brassica pekinensis, Capsicum annuum, Aster scraber, Dipsacus asper, Phyllostachys edulis, Coffea Arabica, Rosa damascene, Vaccinium corybosum, Coriandrum sativum, Teucrium chamaedrys, Lycopersicon esculentum, Triticum aestivum, Acacia nilotica, Aconitum napellus, Adonis vernalis, Allium ampeloprasum, Althea officinalis, Anethum graveolens, Angelica archangelica, Apium graveolens, Arnica Montana, Artemisia abrotanum, Artemisia ansinthium, Beta vulgaris, Boehmeria nivea, Brassica nigra, Calendula officinalis and Elephantopus mollis.

In one embodiment of the present invention the particles furthermore comprise a compound selected from the group consisting of grifolin, grifolic acid, grifolic acid methyl ester, caffeic acid, chlorogenic acid, caffeoyl quinic acid and esters of these acids.

Suitable polymers are cellulose, derivatives of cellulose and the resin shellac. In one embodiment of the present invention the polymer is ethylcellulose. Derivatives of cellulose means modified cellulose, e. g. ethylcellulose and other derivatives of cellulose as described in the online encyclopedia of chemistry **Römpp online, Version 3.6 (printout of "Cellulose-Derivate" of July 14, 2010).**

Particle forming can be done in a one-step drying process or in a two-step or in a multi-step drying process. In a possible one step process the polymer is mixed with an emulsion that contains the nutrient and, optionally, auxiliaries (carrier, emulsifier, etc.). The resulting mixture is dried, e. g. spray dried. Alternatively, a polymer solution or dispersion is spray dried simultaneously with an emulsion that contains the nutrient and, optionally, auxiliaries. The spray drying can be done using a multi nozzle spray dryer.

A possible two (or multi) step process comprises pre-products (powders, granules) that are covered by a polymer in a separate coating step.

Particles according to the present invention can retard the disintegration and therefore release of the nutrient in an (artificial or natural) stomach/intestine to a different degree. The retention time depends (among others parameters) on the kind of polymer used to protect the nutrient, on the amount of polymer used to protect the nutrient and on the production process used to make the particles. Thus the particles allow for tuning the place where the nutrients are released in the intestine.

The amount of polymer in the particles according to the present invention is preferably in the range of 1 - 99 % by weight whereas a more preferred range is 5 - 50 % by weight. The amount of nutrient (GLP-1 stimulating material) in the particles according to the present invention is preferably in the range of 1 ― 99 % by weight. A more preferred range is 10 -70 % by weight.

### Examples

% means % by weight in all the following examples.

### Example 1a (Comparison Example)

Unprotected particles having the following composition were produced.

### Composition of unprotected particles:

| | |
|---|---|
| Oleic acid | 42.86 parts by weight |
| Modified starch (emulsifier) | 47.57 parts by weight |
| Glucose sirup (carrier) | 9.55 parts by weight |

The unprotected particles were produced in the following way. An emulsion containing the oleic acid as nutrient, the emulsifier and the carrier was spray granulated using a Procell 5 spouted bed granulator.

### Example 1b (According to the Present Invention)

Protected particles having the following composition were produced.

### Composition of protected particles:

| | |
|---|---|
| Oleic acid | 30 % |
| Modified starch (emulsifier) | 33.3 % |
| Glucose sirup | 6.7 % |
| Fractionated coconut oil | 5 % |
| Ethylcellulose | 25 % |

The protected particles were produced in the following way. The unprotected particles (granules) that have been produced before were coated using an aqueous ethylcellulose dispersion (Surelease® E-7-19040 from the company Colorcon containing dispersed ethylcellulose and fractionated coconut oil and oleic acid as auxiliaries). The coating was done using a Glatt AGT150 fluid bed coater. The coated granules were tempered at 62 °C to optimize the coating layer.

Both the unprotected particles and the particles protected by the ethylcellulose were tested regarding their resistance against artificial stomach fluid and artificial intestine fluid. Small amounts of samples were taken from the fluids after defined time periods to analyze the release of oleic acid from the particles. This can be done by detecting directly the amount of the oil or by detecting a tracer that was mixed in the oil before the particles where prepared. The release of nutrients in the described examples was detected by analyzing the release of tocopherol which was used as tracer.

The particles were exposed to the stomach fluid for 120 min each and then the fluid containing the particles was converted to intestinal conditions by changing pH and adding the respective enzymes and bile. This converting process lasted not longer than 1 min. The artificial gastrointestinal fluids were prepared according to the publication of Hack et al., Toxicology Letters, 1996; 88:199-210**.**

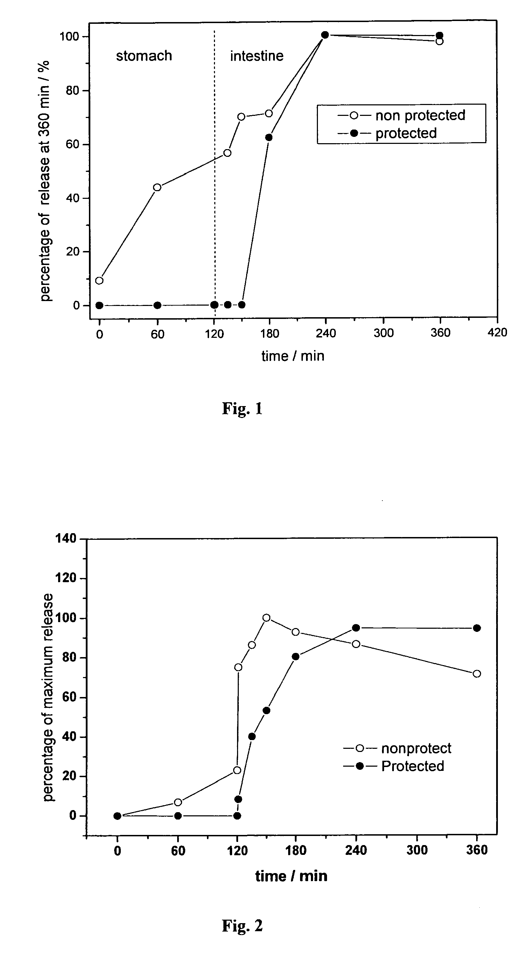

Fig. 1 shows the release pattern of unprotected and protected particles of example 1. Conditions change at 120 min from gastro to intestinal. Fig. 1 shows the release of nutrient for both protected and unprotected particles after being exposed to the artificial gastro-intestinal fluids at t = 0 min. The released amount of nutrients is expressed as percentage of release at t = 360 min which was set as 100 %. The diagram clearly reveals the effect of the polymer on the release of the nutrient. The unprotected particles reach a release rate of 50 % at about 70 min whereas in the case of the protected particles 50 % is released at about 180 min. Additionally, no tocopherol (tracer) could be detected in the first 30 min after the protected particles were exposed to the intestinal conditions.

Typical transition times of food through the human gastrointestinal tract are as follows:
50 % of stomach content emptied after 2.5 - 3 h
Total emptying of the stomach after 4 to 5 h
50 % emptying of the small intestine after 2.5 -3h

This means that the nutrient of the protected particles according to the present invention is mainly released when the particles have passed the stomach and the first section of the small intestine. In contrast to this the unprotected particles begin to release the nutrient immediately after being exposed to the stomach fluid.

### Example 2a (Comparison Example)

Unprotected particles having the following composition were produced.

### Composition of unprotected particles:

| | |
|---|---|
| Oleic acid monogyceride | 50 parts by weight |
| Resistant starch | 37 parts by weight |
| Sodium caseinate | 10 parts by weight |
| Citric esters of mono- and diglycerides | 3 parts by weight |

The unprotected particles were produced in the following way. An emulsion containing all components was spray dried using a multi nozzle dryer from Buechi.

### Example 2b (According to the Present Invention)

Protected particles having the following composition were produced.

### Composition of protected particles:

| | |
|---|---|
| Oleic acid monogyceride) | 34 % |
| Resistant starch | 25.2 % |
| Sodium caseinate | 6.8 % |
| Citric esters of mono- and diglycerides | 2.1 % |
| Ethylcellulose | 24.1 % |
| Fractionated coconut oil | 4.8 % |
| Oleic acid | 3.0 % |

The protected particles were produced in the following way. The particles were produced by spraying the ethylcellulose dispersion (Surelease^{®} E-7-19040 from the company Colorcon containing dispersed a ethylcelluloese and fractionated coconut oil and oleic acid as auxiliaries) simultaneously with the nutrient emulsion (containing carrier material, emulsifier) by means of multi nozzle spray dryer from Buechi.

The resulting particles formed a free flowing powder with a release pattern shown in fig. 2. Fig. 2 shows the release pattern of unprotected and protected particles of example 2. Conditions change at 120 min from gastro to intestinal. The protected particles released 50 % of the oil at about 180 min that is 60 min after being exposed to intestinal conditions. The unprotected particles immediately released the oil after the medium switched to intestinal conditions.

### Example 3 (According to the Present Invention)

### Composition of protected particles:

| | |
|---|---|
| Modified starch | 33.3 % |
| Maltose | 36.7 % |
| Fractionated coconut oil | 5 % |
| Ethylcellulose | 25 % |

## Claims

1. A particle comprising
at least one polymer selected from the group consisting of cellulose, derivatives of cellulose and shellac and
at least one nutrient selected from the group consisting of fatty acids, monoacylglycerols, diacylglycerols, triacylglycerols, proteins and carbohydrates,
wherein the at least one nutrient is coated by the at least one polymer or wherein the at least one nutrient is matrix-encapsulated by the at least one polymer.

2. The particle according to claim 1, wherein the at least one polymer is ethylcellulose.

3. The particle according to claim 1 or 2, wherein the at least one nutrient is selected from the group consisting of fatty acids, monoacylglycerols of fatty acids, diacylglycerols of fatty acids and triacylglycerols of fatty acids.

4. The particle according to any of claims 1 to 3 comprising
1 - 99 % by weight of the at least one polymer and
1 - 99 % by weight of the at least one nutrient.

5. The particle according to any of claims 1 to 3 comprising
5 - 50 % by weight of the at least one polymer and
10 - 70 % by weight of the at least one nutrient.

6. The particle according to any of claims 1 to 5, wherein the particle has a size of less than 1 mm, preferably less than 0.5 mm.

7. The particle according to any of claims 1 to 6 furthermore comprising one or more of a carrier and an emulsifier.

8. A process for making the particle according to any of claims 1 to 7 comprising mixing the at least one polymer with an emulsion that comprises the at least one nutrient and spray drying the resulting mixture.

9. A process for making the particle according to any of claims 1 to 7 comprising forming a first particle comprising the at least one nutrient and
coating this first particle with the at least one polymer.

10. A food product comprising particles according to any of claims 1 to 9, wherein the food product is preferably selected from the group consisting of a dietary supplement (preferably a gelatine capsule) and of a functional food (preferably a dairy product or a beverage) and wherein the functional food preferably contains 0.01 to 10, more preferably 0.1 to 5, more preferably 1 to 3 % by weight of the particles.

11. A medicament comprising particles according to any of claims 1 to 9 further comprising pharmaceutically acceptable additives.

12. The non-therapeutic use of particles according to any of claims 1 to 7 or of the food product according to claim 10 for reducing appetite of a human or for increasing satiety of a human or for reducing body weight of a human.

13. The particles according to any of claims 1 to 7, optionally comprised in a composition further comprising pharmaceutically acceptable additives, for use as a medicament.

14. The particles according to any of claims 1 to 7 or the medicament according to claim 11 for use in the therapeutic or prophylactic reduction of the body weight of a human or for use in the therapeutic or prophylactic treatment of obesity.
